# EUROPÄISCHE PATENTSCHRIFT

(11) **EP 2 282 685 B1**
(45) Veröffentlichungstag und Bekanntmachung des Hinweises auf die Patenterteilung: **27.06.2012**
(21) Anmeldenummer: 09745576.0
(22) Anmeldetag: 15.05.2009
(51) Int. Cl.: A61B 17/16, B23B 45/00

(54) **BOHRVORRICHTUNG, INSBESONDERE KNOCHENBOHRVORRICHTUNG, MIT ANHALTEEINRICHTUNG**
DRILLING DEVICE, IN PARTICULAR BONE DRILLING DEVICE, COMPRISING A STOPPING MECHANISM
DISPOSITIF DE PERÇAGE, NOTAMMENT DISPOSITIF DE PERÇAGE D'OS COMPORTANT UN SYSTÈME D'ARRÊT

(30) Priorität: 16.05.2008 DE 102008023920; 16.05.2008 DE 202008006680 U; 11.07.2008 DE 102008032704
(43) Veröffentlichungstag der Anmeldung: 16.02.2011
(73) Patentinhaber: Huber, Andreas, 84416 Taufkirchen/Vils (DE)
(72) Erfinder: Huber, Andreas, 84416 Taufkirchen/Vils (DE)
(74) Vertreter: Gerber, Wolfram
(86) Internationale Anmeldenummer: PCT/EP2009/003476
(87) Internationale Veröffentlichungsnummer: WO 2009/138242

(56) Entgegenhaltungen:
- DE-A- 10 129 948
- DE-A-102006 006 988
- US-A- 4 456 010
- US-A1- 2007 196 784

## Beschreibung

Die vorliegende Erfindung betrifft eine Bohrvorrichtung, insbesondere Knochenbohrvorrichtung, mit zwei konzentrischen, axial gegeneinander verschieblichen, Teilen, wobei das Innenteil gegenüber dem als Bohrer ausgebildeten Außenteil axial in Bohrrichtung vorgespannt ist und ohne Gegenbeaufschlagung gegenüber dem Außenteil in Bohrrichtung etwas vorsteht, und einer Einrichtung, die bei einer axialen Verschiebung des Innenteils gegenüber dem Außenbohrer den Antrieb des Gesamtbohrers unterbricht.

Eine derartige gattungsgemäße Vorrichtung ist z. B. aus der US-PS 4 456 010 bekannt. In der Praxis des Bohrens, insbesondere beim Knochenbohren, stellt sich häufig das Problem, dass in einer unbekannten Tiefe sich der Zustand des durchbohrten Raumes ändert. Das ist schon jedem Heimwerker bekannt, der bei einer Wand unbekannter Dicke plötzlich im Hohlraum dahinter landet, wobei der Bohrer letztendlich durch den Anschlag des Bohrfutters am weiteren Vordringen gehindert wird. Beim Knochenbohren ist dies besonders gefährlich, weil der Bohrer beim unkontrollierten Durchbrechen des (harten) Knochen die dahinterliegenden weichen Gewebe (Schleimhäute, Hirnhaut) sozusagen aufwickelt und zerstört. Bei der bekannten Vorrichtung wird dies dadurch verhindert, dass beim Durchbrechen der Innenbohrer gegenüber dem Außenbohrer mit etwas größerem Durchmesser durch die axiale Vorspannung vorrückt und dadurch eine Kupplung ausrückt, die beim Ansetzen des Bohrers durch eine Rückverschiebung gegen die axiale Vorspannung in Eingriff gelangt war. Das Ausrücken der Kupplung hält dann den Antrieb und die Bohrer an.

Aus obigem wird die Wichtigkeit einer schnellen und zuverlässigen Reaktion der Einrichtung deutlich. Dies ist bei der Lösung im Stande der Technik nicht immer gewährleistet, da die Auslösung der Kupplung mit einem Zeitverlust einhergeht und die Kupplungsmechanik auch störanfällig, etwa durch eingedrungenes Bohrgut, ist. Zudem dreht der Bohrer nachteilig aufgrund seiner gespeicherten kinetischen noch unkontrolliert nach dem Auskuppelvorgang weiter.

Aus der DE 10129948 ist ebenfalls eine Knochenbohrvorrichtung bekannt, bei der ein zweiteiliger Bohrer verwendet wird, dessen Spitze immer dann, wenn sie auf ein weniger dichtes Medium stößt, weiter nach vorne dringt als der übrige Bohrkörper und von der Drehbewegung entkoppelt werden kann. Die Bohrvorrichtung hat ein entsprechendes Auswertesystem, welches die Spitze und deren Relativbewegung gegenüber dem übrigen Bohrkörper erkennt und den sofortigen Stopp der Drehbewegung durch Unterbrechung der Motorstromzufuhr und Abbremsen veranlasst. Die Spitze wird mittels eines Fluids nach vorne in Richtung Bohrstelle druckbeaufschlagt. Sobald die Spitze in ein weicheres Medium eindringt und somit dem übrigen Rohrkörper voreilt, sinkt der Druck aufgrund der Volumenvergrößerung der Fluidzuführkammer. Der Druck wird mittels eines Drucksensors, welcher außerhalb des Handgriffs für die Bohrvorrichtung angeordnet ist, überwacht und an die Steuereinrichtung der Bohrvorrichtung übermittelt, welche das Abbremsen und Abschalten des Antriebes der Bohrvorrichtung steuert. Nachteilig bei dieser Bohrvorrichtung ist, dass teure Drucksensoren verwendet werden müssen, die geringste Druckunterschiede messen können, da die Relativbewegung geringer als 1 mm ist. Zudem können Druckschwankungen bei der Erzeugung des erforderlichen Fluiddrucks entstehen, die eine eindeutige Erkennung des Durchbruchs der Bohrerspitze in ein weicheres Medium erschweren wenn nicht sogar unmöglich machen. Auch sind zusätzliche aufwendige Maßnahmen zur Anordnung und Abdichtung des Fluidsystems erforderlich. Der Einsatz einer Fluidpumpe ist zudem teuer und wartungsanfällig. Eine Bohrvorrichtung mit den Merkmalen des Oberbegriffs des Anspruchs 1 ist aus DE-A- 102006006988 bekannt.

Der Erfindung liegt die Aufgabe zugrunde, eine gattungsgemäße Bohrvorrichtung derart weiter zu bilden, dass sie schneller und zuverlässiger anhält und insbesondere auch einen wesentlich einfacheren und dadurch leichter und preisgünstiger herstellbaren Aufbau hat.

Die Aufgabe wird erfingdungsgemäß mit einer Bohrvorrichtung mit den Merkmalen des Anspruchs 1 gelöst. Weitere vorteilhafte Ausgestaltungen ergeben sich durch die Merkmale der auf den Anspruch 1 rückbezogenen Ansprüche.

Der Erfindung liegt der Gedanke zugrunde, die Relativbewegung direkt mittels eines Sensors zu erfassen, wodurch der Antrieb von der Steuereinrichtung der Bohrvorrichtung abgeschaltet oder abgebremst wird. Der Sensor ist dabei vorteilhaft direkt hinter oder neben dem hinteren Ende des Außenteil und/oder Innenteils des Bohrers, insbesondere im Gehäuse des Handgriffs angeordnet. Hierdurch wird eine platzsparende Anordnung erreicht, die ein gutes Handling z.B. im Mundraum eines Patienten garantiert.

Die Erfindung hat insbesondere folgende Vorteile: Durch die elektromagnetische Auslösung (diese kann z. B. bevorzugt kapazitiv, alternativ induktiv, weiter alternativ durch eine Lichtschranke sein) kann schon eine minimale Axialverschiebung des Innenteils (in der Praxis z. B. 0,3 bis 0,5 mm) die Schaltung auslösen und den Anhalte- und ggf. Bremsvorgang auslösen. Mit Hilfe einer Sensorlösung lässt sich der Aufbau auch erheblich einfacher und kompakter gestalten, wie nachfolgend noch dargelegt werden wird.

Bevorzugt wird der Schaltvorgang durch das den Außenbohrer rückwärtig in Axialrichtung überragende Ende des Innenteils, das einem Sensor gegenüberliegt, ausgelöst. Die Bewegung des Innenteils ist dann eine reine Gleitbewegung, die durch entsprechende Materialien und/oder Beschichtungen reibungsarm gehalten werden kann und sonst keine mechanische Arbeit leisten muss. Durch die Anordnung des Sensors gegenüber den rückwärtigen Enden der Bohrer kann deren Gestaltung auch einfach gehalten werden.

Bevorzugt ist das Ende des Innenteils radial, d.h. kragenförmig, erweitert. Diese Erweiterung kann z. B. in Gestalt einer - leicht an das Ende anzuformenden oder anzubringenden Scheibe ausgebildet sein. Dadurch wird die Wirkfläche für eine kapazitive oder induktive Beeinflussung des Sensors vergrößert, der dadurch entsprechend feiner eingestellt werden kann.

Bevorzugt kann das den Außenbohrer rückwärtig in Axialrichtung überragende Ende des Innenbohrers, einen Sensor beaufschlagenden Lichtstrahl freigeben bzw. sperren, wodurch die Abschaltung ausgelöst werden kann. Auch diese Lösung lässt sich einfach und außerhalb des Gesamtbohrers, bestehend aus Außen- und Innenteil, verwirklichten.

Bevorzugt ist das Innenteil als im wesentlichen zylindrischer Stift ausgebildet. Dies hat insbesondere den Vorteil, dass der Stift, ohne selbst eine Bohrfunktion zu haben, wodurch er noch günstiger herstellbar ist, bei entsprechender Schmalheit leicht durch den von der umgebenden Bohrspitze des Außenbohrers geschaffenen, anfänglich noch minimalen, Durchbruch hindurchtreten und den Gesamtbohrer anhalten kann.

Das in Bohrrichtung liegende Ende des Innenteils ist als Bohrspitze mit entsprechenden Schneiden ausgebildet. Dies hat insbesondere den Vorteil, dass die beiden Bohrer eine gemeinsame Bohrspitze bilden können und eine bessere Abfuhr des Bohrgutes gewährleistet ist.

Der Außenbohrer und das Innenteil sind zueinander drehfest. Dies hat insbesondere den Vorteil, dass kein Bohrgut durch eine Relativdrehung zwischen den Bohrer geraten kann.

Bevorzugt wird die drehfeste Verbindung von Innenteil und Außenbohrer durch einen senkrecht zur Bohrachse angeordneten, durch das Innenteil und zwei Langlöcher im Außenbohrer, deren Länge den Hub des Innenteils zum Außenbohrer bestimmt, gehenden Zylinderstift geschaffen. Auf diese Weise lassen sich die beiden Teile bzw. Bohrer besonders einfach miteinander drehverbinden, und vor allem lassen sich durch die Entfernung des Stifts beide Teile leicht trennen. Der Stift ist auch in der Lage, im Zusammenwirken mit zwei Langlöchern im Mantel des Außenbohrers die Axialbewegung des Innenteils zu begrenzen, das sich sonst unter der Vorspannung u. U. unerwünscht weit aus dem Außenbohrer bewegen könnte.

Bevorzugt wird die drehfeste Verbindung von Innenteil und Außenbohrer durch an der Außenseite des Innenteils und der Innenseite des Außenbohrers radial verlaufende Nuten und Stege, die miteinander durch radiales Stecken in Eingriff bringbar sind, geschaffen.

Dadurch, dass die Nuten zumindest in einer Richtung axial begrenzt sind, kann auch ein Anschlag für den Vortritt des Innenteils geschaffen werden.

Die beiden Bohrer bilden in der rückwärtigen Lage des Innenbohrers eine gemeinsame Bohrspitze. In der Riegel ist das Verhältnis der beiden Bohrer durch Anschläge (u. B. die Längsnuten oder die Langlöcher) begrenzt. Beim Ansetzen des Bohrers wird das Innenteil bis an den Vorderrand des Außenteils zurückgedrängt, wodurch optional auch der Antrieb der Bohrvorrichtung eingeschaltet werden kann. Die jeweilige Spitze bzw. Kegelstumpfspitze wirken dann zweckmäßig wie ein einheitlicher Bohrer. Dadurch wird auch eine bessere Abfuhr der Bohrspäne, insbesondere durch den Innenbohrer bzw. das Innenteil, gefördert.

In der rückwärtigen Lage des Innenbohrers fluchten die Schneiden der beiden Bohrer in radialer Richtung. Dadurch wird die Wirkung einer einheitlichen Bohrspitze gefördert.

Bevorzugt wird die axiale Vorspannung des Innenteils durch eine, zwischen einem Außenflansch des Innenteils und einem Innenflansch des Außenbohrers eingespannte Feder bewirkt. Dies ist eine einfache und zuverlässige Lösung, und die Feder kann außerhalb, nämlich rückwärtig, des eigentlichen Bohrbereichs und damit diesen nicht radial erweiternd, angebracht werden.

Bevorzugt ist das als Innenbohrers ausgebildete Innenteil in seinem ausgefahrenen Zustand fixierbar, z. B. per Tastendruck. Dadurch kann es, in an sich bekannter Weise, als Zentrierbohrer dienen.

Bevorzugt findet die Bohrvorrichtung ihre Anwendung in der Zahnmedizin. Dort ist die, durch die erfindungsgemäße Lösung mögliche kleine Bauform von besonderem Vorteil.

Nachfolgend wird die Erfindung anhand von Zeichnungen, auf die wegen ihrer großen Klarheit und Übersichtlichkeit hinsichtlich der Offenbarung ausdrücklich verwiesen wird, näher beschrieben.

Es zeigen:
- Figur 1:: Eine schematische Seitenansicht einer bevorzugten Ausführungsform einer erfindungsgemäßen BohrvorRichtung, in der der Innenteil im Außenbohrer erkennbar ist, mit dem Sensor und dem Antrieb;
- Figur 2:: den Innenteil und den Außenbohrer der Erfindung, wobei der Innenteil entgegen der Vorspannung durch eine Druckfeder nach rückwärts belastet ist;
- Figur 3:: den Innenteil und den Außenbohrer der Erfindung, wobei der Innenteil unter der Vorspannung durch eine Druckfeder aus dem Außenbohrer weitestmöglich ausgefahren ist;
- Figur 4:: eine alternative Ausführungsform der erfindungsgemäßen Bohrvorrichtung mit Kegelradantrieb.

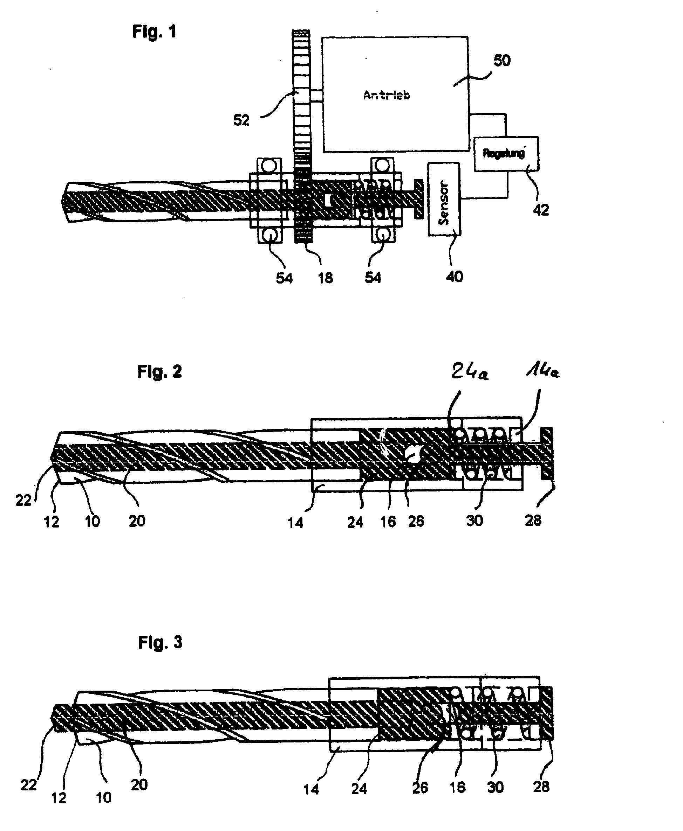

In Figur 1 ist der Gesamtbohrer dargestellt, der einen Außenbohrer 10 (durchscheinend gezeigt) und ein darin radial verschiebliches, als Innenbohrer ausgebildetes und schraffiert dargestelltes, Innenteil (20) aufweist. Der Außenbohrer 10 weist einen Zahnkranz 18 auf, der wiederum durch ein Zahnrad 52, das auf einem Antrieb 50 sitzt, angetrieben wird. Der Antrieb dreht beide Bohrer 10, 20 des Gesamtbohrers, weil diese durch einen durchgehenden Zylinderstift 26 drehfest miteinander verbunden sind. Der Außenbohrer 10 ist in einer Drehlagerung 54 gelagert, welche nicht im Einzelnen dargestellt ist. Für bestimmt Anwendungen soll es allerdings nicht ausgeschlossen sein, dass Außenbohrer und Innenbohrer getrennt angetrieben werden. Der Innenbohrer 20 hat ein erweitertes Ende 28, das einem Sensor 40 gegenüberliegt. Der Sensor 40 ist so eingestellt, dass er bei einer einem Abstand x entsprechenden Stellung des erweiterten Endes 28 der Steuerung 42 mitteilt, dass der Antrieb 50 laufen soll, der somit dann, wenn er nicht durch einen anderen Schalter in Reihe ausgestellt ist, die Gesamtbohrer 10 und 20 antreibt. Die Steuerung 42 kann auch weiter ausgebildet sein, z. B. kann sie die Drehgeschwindigkeit in Abhängigkeit von der Bohrtiefe ändern usw. Die Figur 2 zeigt den Innenbohrer 20 und den Außenbohrer 10 in der Stellung, wie in Figur 1, die sie zueinander einnehmen, wenn der Gesamtbohrer auf das zu durchbohrende Material aufgesetzt ist und somit der Innenbohrers 20 gegen die Kraft der Druckfeder 30 in das Innere des Außenbohrers 10 gedrängt wird. Der Stift 26 schlägt dann an das passend gewählte hintere Ende des Langlochs 16 im Mantel des Außenbohrers 10 an.

Die Figur 3 zeigt die Stellung, wenn der Innenbohrer 10 durch das Ende des harten Materials, z. B. des Knochens, durchgebrochen ist. Am Beginn des Durchbruchs aus einem harten Bereich (am Ende des Hartteils) wird der Durchmesser, abhängig von Schneidwinkel und Vorschub, kontinuierlich größer. Bei Erreichen des Durchmessers des Innenbohrers 20 an dieser, der Durchbruchsstelle, tritt dieser Innenbohrer 20 mittels der vorzugsweise einstellbaren Federkraft des Federelementes 30 axial in Richtung Bohrerschneide 22 (Bohrrichtung) aus dem Außenbohrer 10 hervor. Durch diese axiale Positionsverschiebung zum Außenbohrer 10, welche der Sensor 40 erkennt (im Ausführungsbeispiel ist als Abstandsänderung, die ein Sensorsignal auslöst, 0,3 mm gewählt, was ohne weiteres erreichbar ist), wird das abrupte Anhalten der Drehung der Bohreinheit bzw. des Bohrers ausgelöst, so dass keinerlei Beschädigungen durch rotierende Werkstücke an Weichteilen erfolgen können.

Durch die Erfindung ist es möglich, den Bohrer so schnell anzuhalten, dass der Anhaltevorgang von Betriebsdrehzahl zu Stillstand innerhalb von ca. 0,9° einer Umdrehung des Bohrers beendet ist. Somit ist die Gefahr der Verletzung von Weichteilen, wie z. B. Nerven oder Blutgefäßen, stark verringert.

Dabei rückt die Bohrspitze 22 des Innenbohrers 20 vor und das erweiterte Ende 28 des Innenbohrers 20 rückt vom Sensor 40 (der einerseits einen festen Abstand vom Ende des Außenbohrers 10 hat) in Bohrrichtung unter der Federkraft der Feder 30 ab. Der Innenbohrer 10 wird dabei vermittels des Halters 24 in der Axialführung 14 beführt. Ab einem vorgewählten, einstellbaren Abstand des erweiterten Endes 28 vom Sensor 40 schaltet der Sensor 40 über die Steuerung 42 den Antrieb aus, der so eingerichtet ist, dass er sofort anhält. Dies wird auch mit dadurch erreicht, dass als Antrieb ein Schrittmotor gewählt wird. Das Anhalten kann mit einer besonderen Ansteuerelektronik 42 im Achtelschrittbetrieb bereits bei 0,113° geschehen. Ein zu weites Vorrücken des Innenbohrers 20 gegenüber dem Außenbohrer 10 wird wiederum durch den Anschlag des Zylinderstiftes 26, diesmal am vorderen Ende des Langlochs 16, verhindert.

Eine geeignete Ausbildung der Schneiden 22 des Innenbohrers 20 (Winkel, Schärfe usw.) die zweckmäßig nach dem zu bohrenden Material gewählt wird, kann verhindern, dass der axial bewegliche und unter Federspannung stehende Innenbohrer 20 dem Außenbohrer 10 schon vor dem Durchbruch voreilt.

Wie gesagt hat der erfindungsgemäße Aufbau auch den Vorteil, dass der Innenbohrer, z. B. durch Fixierung in seiner Vorderstellung, als Zentrierbohrer genutzt werden kann.

Die erfindungsgemäße Bohrvorrichtung kann auch verwendet werden, wenn bei der Durchbohrung eines weicheren Materials das Anbohren eines härteren Materials (z. B. einer eingepflanzten Titanplatte) verhindert werden soll. Dabei wird die Bohrstellung der beiden Bohrer zueinander, z. B. durch eine stärkere Feder 30', so gewählt, dass der Innenbohrer 20 wie in Figur 3 ersichtlich, dem Außenbohrer 10 von vornherein voreilt. Beim Anstoßen an ein härteres Material wird der Innenbohrer nun gegen die Feder 30' zurückgedrängt, so dass er sich dem Sensor 40 nähert. Das Signal des Sensors an die Steuerung muss dann einfach so umgepolt oder von der Regelung 42 so umgedeutet werden, dass in diesem Fall eine vorwählbare Annäherung (z. B. wieder eine Veränderung um 0,3 mm) den Stillstand des Antriebs 50 auslöst.

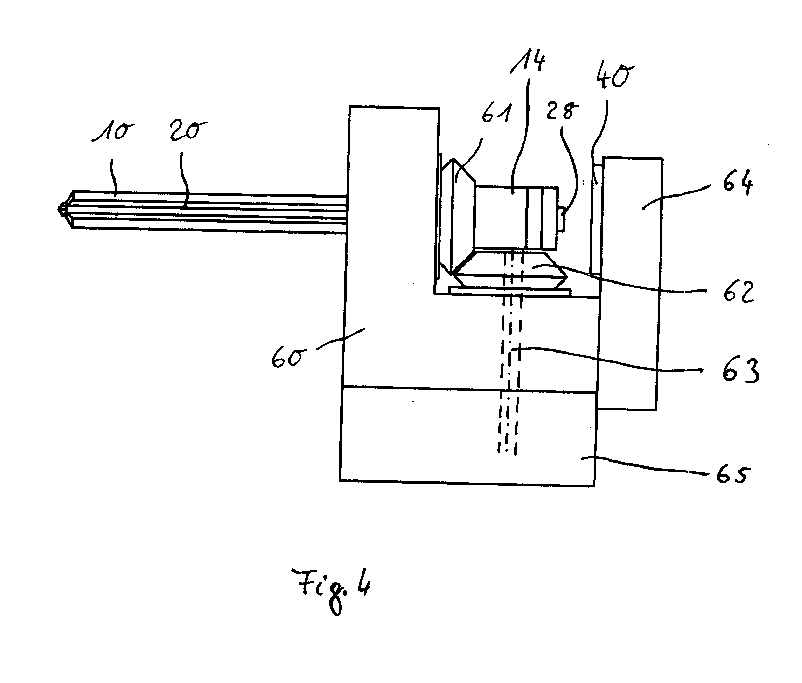

Die Figur 4 zeigt eine alternative Ausführungsform der erfindungsgemäßen Bohrvorrichtung mit Kegelradantrieb. Der Außenbohrer 10 weist an seinem hinteren Ende einen Bereich 14 mit größerem Durchmesser auf, an dem das erste Kegelrad 61 drehfest befestigt ist. Das erste Kegelrad 61 zahnt mit dem zweiten Kegelrad 62, welches auf einer Welle 63 befestigt ist, welche mit einem nicht dargestellten Antrieb in Verbindung ist. Der Außenbohrer 10 sowie die welle 62 sind in dem Gehäuseteil 60 gelagert, an dem ein weiterer Bereich 64 befestigt oder angeformt ist, welcher den Sensor 40 trägt. Der Sensor 40 ist in axialer Richtung hinter dem Ende 28 des Innenteils 10 und im Abstand zu diesem angeordnet. An dem Gehäuseteil 60 kann ein weiteres Gehäuseteil 65 zur Aufnahme des Antriebs 50 und/oder der Regelungseinrichtung 42 befestigt sein.

## Patentansprüche

1. Bohrvorrichtung, insbesondere Knochenbohrvorrichtung, mit zwei konzentrischen (20, 10) axial gegeneinander verschieblichen Teilen (20, 10), wobei das Innenteil (20) gegenüber dem als Bohrer ausgebildeten Außenteil (10) axial in Bohrrichtung mittels einer Feder (30) vorgespannt ist und ohne oder bei zu geringer Kraftgegenbeaufschlagung mit seiner Spitze (22) gegenüber der Spitze (12) des Außenteils (10) in Bohrrichtung etwas vorsteht, **dadurch gekennzeichnet, dass** das in Bohrrichtung liegende Ende des Innenteils (20) als Bohrspitze (22) mit entsprechenden Schneiden ausgebildet ist, und dass in der rückwärtigen Lage des Innenteils (20) dieses gemeinsame mit dem Außenteil (10) eine gemeinsame Bohrspitze bildet und die Schneiden vom Innenteil (20) und Außenteils (10) zueinander in radialer Richtung fluchten und einer Einrichtung, die bei einer axialen Verschiebung des Innenteils (20) gegenüber dem Außenbohrer (10) den Antrieb des Gesamtbohrers (20, 10) unterbricht, wobei das Innenteil (20) zum Außenteil (10) drehfest ist, und ein Sensor (40) in einem festen Abstand relativ zum Ende des Außenteils (10) angeordnet ist und die axiale Verschiebung des Innenteils (20) zum Außenteil (10), insbesondere kapazitiv, induktiv oder mittels Lichtschranke, detektiert und die Einrichtung bei Verschiebung des Innenteils (20) gegenüber dem Außenteil (10) um einen vorgegebenen Wert den Antrieb (50) des Bohrers (20, 10), insbesondere unmittelbar, anhält.

2. Bohrvorrichtung nach Anspruch 1, **dadurch gekennzeichnet, dass** die Relativbewegung des den Außenbohrer (10) rückwärtig in Axialrichtung überragenden Endes (28) des Innenteils (20) gegenüber dem Sensor (40) den Schaltvorgang zum Anhalten des Antriebs (50) auslöst.

3. Bohrvorrichtung nach Anspruch 2, **dadurch gekennzeichnet, dass** das Ende (28) des Innenteils (20) radial, insbesondere in Form eines Kragens, erweitert ist.

4. Bohrvorrichtung nach Anspruch 1, **dadurch gekennzeichnet, dass** der Schaltvorgang durch das den Außenbohrer (10) rückwärtig in Axialrichtung überragende Ende (28) des Innenbohrers (20), das einen, einen Sensor beaufschlagenden, Lichtstrahl freigibt bzw. sperrt, ausgelöst wird.

5. Bohrvorrichtung nach einem der Ansprüche 1 bis 4, **dadurch gekennzeichnet, dass** das Innenteil (20) als im wesentlichen zylindrischer Stift ausgebildet ist.

6. Bohrvorrichtung nach einem der vorhergehenden Ansprüche, **dadurch gekennzeichnet, dass** die axiale Vorspannung des Innenteils (10) durch eine, zwischen einem Außenflansch (24a) des Innenteils (20) und einem Innenflansch (14a) des Außenbohrers (10) eingespannte Feder (30) erfolgt.

7. Bohrvorrichtung nach einem der vorhergehenden Ansprüche, **dadurch gekennzeichnet, dass** der Innenbohrer (20) in seinem ausgefahrenen Zustand fixierbar ist.

8. Bohrvorrichtung nach einem der vorhergehenden Ansprüche, **dadurch gekennzeichnet, dass** der Sensor (40) ein Hall-Sensor ist, der das Ende (28) des Innenteils (20) detektiert.

9. Bohrvorrichtung nach einem der vorhergehenden Ansprüche, **dadurch gekennzeichnet, dass** der Sensor (40) ein Sensorsignal erzeugt, welches ein Eingangssignal der Einrichtung ist und anhand dessen die Einrichtung ein Schaltmittel, insbesondere einen Transistor oder ein Relais, zum Abschalten des Antriebs (50) ansteuert.

10. Bohrvorrichtung nach einem der vorhergehenden Ansprüche, **dadurch gekennzeichnet, dass** der Sensor (40) neben oder axial hinter dem Ende (28) des Innenteils (20) im Handgriff der Bohrvorrichtung, angeordnet ist,

11. Bohrvorrichtung nach einem der vorhergehenden Ansprüche, **dadurch gekennzeichnet, dass** der vorgegebene Wert mittels eines Eingabemittels, insbesondere in Form eines Potentiometers oder einer Tastatur, vorgebbar und/oder einprogrammierbar ist.

12. Bohrvorrichtung nach einem der vorhergehenden Ansprüche, **dadurch gekennzeichnet, dass** das Außenteil (10) mit einem ersten Kegelrad drehfest verbunden ist, welches mit einem zweiten Kegelrad verzahnt ist, wobei das zweite Kegelrad mit einem ersten Ende einer, insbesondere biegsamen, Welle verbunden ist, die mit ihrem anderen zweiten Ende mit dem elektrischen Antrieb (50) oder einem dazwischen geschalteten Getriebe verbunden ist.

13. Bohrvorrichtung nach Anspruch 13, **dadurch gekennzeichnet, dass** das erste Kegelrad das Außenteil umfasst oder an dem Ende des Außenteils befestigt ist, wobei das zweite Kegelrad an dem Handgriff oder einem darin angeordneten Teil gelagert ist.

14. Bohrvorrichtung nach einem der vorhergehenden Ansprüche, **dadurch gekennzeichnet, dass** der Sensor (40) ein Abstandssensor oder Entfernungesensor ist, der die Entfernung des Endes (28) des Innenteils (20) zum 5 Sensor (40) ermittelt und ein entsprechendes Signal erzeugt und an die Einrichtung übergibt.

15. Bohrvorrichtung nach einem der vorhergehenden Ansprüche, **dadurch gekennzeichnet, dass** durch Kraftbeaufschlagung der Spitze (22) des Innenteils (20) entgegen der Federkraft der Feder (30) in Richtung Sensor (40) verstellbar ist, wodurch die Einrichtung den Antrieb (50) startet.

16. Bohrvorrichtung nach einem der vorhergehenden Ansprüche, **dadurch gekennzeichnet, dass** die Re-5 gelungseinrichtung (42) den Antrieb ausschaltet, sobald der Sensor (40) eine Relativbewegung von genügender Größe detektiert.

17. Bohrvorrichtung nach Anspruch 17, **dadurch gekennzeichnet, dass** die Relativbewegung durch ein plötzliches Eindrücken der während des Bohrvorgangs vorauseilenden Zentrierspitze (22) des Innenteils (20) aufgrund einer äußeren Gegenkraft, die größer als die Federkraft der Feder (30) ist, erfolgt.

18. Bohrvorrichtung nach einem der vorhergehenden Ansprüche, **dadurch gekennzeichnet, dass** die drehfeste Verbindung von Innenteil (20) und Außenbohrer (10) entweder
- durch einen senkrecht zur Bohrachse angeordneten, durch das
Innenteil (10) und zwei Langlöcher (16) im Außenbohrer (10), deren Länge den Hub des Innenteils (20) relativ zum
Außenbohrer (10) bestimmt, gehenden Zylinderstift (26) erfolgt
- oder durch an der Außenseite des Innenteils (20) und der
Innenseite des Außenbohrers (10) radial verlaufende Nuten
und Stege, die miteinander durch radiales Stecken in Eingriff bringbar sind, bedingt ist.

## Claims

1. Drilling device, in particular a bone drilling device, comprising two concentric parts (20, 10) that can be axially moved towards each other, wherein the inner part (20) is axially preloaded relative to the outer part (10) designed as a drill in the drilling direction by means of a spring (30) and the tip (22) of the inner part (20) slightly protrudes relative to the tip (12) of the outer part (10) in the drilling direction when no or too little force is applied, **characterised in that** the end of the inner part (20) lying in the drilling direction is designed as a drill bit (22) with corresponding cutting edges, and **in that** the rear position of the inner part (20) of this together with the outer part (10) forms a joint drill bit and the cutting edges of the inner part (20) and outer part (10) are aligned with one another in the radial direction, and a mechanism which interrupts the driving operation of the entire drill (20, 10) when the inner part (20) is axially moved relative to the outer drill (10), wherein the inner part (20) is rotationally fixed relative to the outer part (10) and a sensor (40) is arranged at a set distance from the end of the outer part (10) and detects the axial movement of the inner part (20) towards the outer part (10), especially in a capacitive or inductive manner or by means of a photoelectric barrier and the mechanism in particular directly stops the drive unit (50) of the drill (20, 10) when the inner part (20) is moved by a predefined value relative to the outer part (10) .

2. Drilling device according to claim 1, **characterised in that** the movement of the end (28) of the inner part (20) protruding backwards in the axial direction beyond the outer drill (10) relative to the sensor (40) triggers the switching operation to stop the drive unit (50).

3. Drilling device according to claim 2, **characterised in that** the end (28) of the inner part (20) is radially expanded, in particular in the form of a collar.

4. Drilling device according to claim 1, **characterised in that** the switching device is triggered by the end (28) of the inner drill (20) protruding backwards in the axial direction beyond the outer drill (10), which releases or blocks a light beam impinging upon the sensor.

5. Drilling device according to one of claims 1 to 4, **characterised in that** the inner part (20) is essentially designed as a cylindrical pin.

6. Drilling device according to one of the above claims, **characterised in that** the axial preloading of the inner part (10) is achieved through a stressed spring (30) between an outer flange (24a) of the inner part (20) and an inner flange (14a) of the outer drill (10).

7. Drilling device according to one of the above claims, **characterised in that** the inner drill (20) is fixable in its extended position.

8. Drilling device according to one of the above claims, **characterised in that** the sensor (40) is a Hall sensor, which detects the end (28) of the inner part (20).

9. Drilling device according to one of the above claims, **characterised in that** the sensor (40) generates a sensor signal which is an input signal to the mechanism and by means of which the mechanism activates a switching means, in particular a transistor or a relay, for stopping the drive unit (50).

10. Drilling device according to one of the above claims, **characterised in that** the sensor (40) is arranged alongside or axially behind the end (28) of the inner part (20) in the handle of the drilling device.

11. Drilling device according to one of the above claims, **characterised in that** the specified value can be specified and/or programmed by means of an input means, in particular in the form of a potentiometer or a keyboard.

12. Drilling device according to one of the above claims, **characterised in that** the outer part (10) is secured against rotation to a first bevel gear, which meshes with a second bevel gear, wherein the second bevel gear with a first end is connected with an in particular flexible shaft, which with its other second end is connected with the electrical drive unit (50) or a gear unit connected in between.

13. Drilling device according to claim 13, **characterised in that** the first bevel gear comprises the outer part or is secured to the end of the outer part, wherein the second bevel wheel is supported by the handle or a part arranged therein.

14. Drilling device according to one of the above claims, **characterised in that** the sensor (40) is a distance sensor or a proximity sensor, which determines the distance between the end (28) of the inner part (20) and the sensor (40) and generates the corresponding signal to the device.

15. Drilling device according to one of the above claims, **characterised in that** the tip (22) of the inner part (20) can be adjusted against the spring force of the spring (30) in the direction of the sensor (40), wherein the device starts the drive unit (50).

16. Drilling device according to one of the above claims, **characterised in that** the adjusting device (42) stops the drive unit as soon as the sensor (40) detects a relative movement of sufficient magnitude.

17. Drilling device according to claim 17, **characterised in that** the relative movement takes place by the sudden pushing-in of the centring tip (22) of the inner part (20) running ahead during the drilling process due to an external opposing force that is greater than the spring force of the spring (30).

18. Drilling device according to one of the above claims, **characterised in that** the connection secured against rotation of the inner part (20) and the outer drill (10) either
- is achieved by a cylindrical pin (26) arranged vertically to the drilling axis, passing through the inner part (10) and two slotted holes (16) in the outer dill (10), the length of which determines the travel of the inner part (20) relative to the outer drill (10);
- or is determined by grooves and webs running radially on the outside of the inner part (20) and the inside of the outer drill (10) which can be brought into engagement which each other through radial interlocking.

## Revendications

1. Dispositif de perçage, en particulier dispositif de perçage pour os, comprenant deux parties concentriques (20, 10) déplaçables axialement l'une par rapport à l'autre, telles que la partie intérieure (20) est précontrainte par rapport à la partie extérieure (10), réalisée sous forme d'outil de perçage, axialement dans la direction de perçage au moyen d'un ressort (30) et, en l'absence d'une force antagoniste ou en présence d'une force antagoniste trop faible, dépasse légèrement en direction de perçage avec sa pointe (22) par rapport à la pointe (12) de la partie extérieure (10),
**caractérisé en ce que** l'extrémité, située en direction de perçage, de la partie intérieure (20) est réalisée sous forme de pointe de perçage (22) avec des tranchants correspondants, et **en ce que** dans la position postérieure de la partie intérieure (20) celle-ci forme, conjointement avec la partie extérieure (10), une pointe de perçage commune, et les tranchants de la partie intérieure (20) et de la partie extérieure (10) sont mutuellement en alignement en direction radiale, et comprenant un système qui, lors d'un déplacement axial de la partie intérieure (20) par rapport à la partie extérieure (10), interrompt l'entraînement de la totalité de l'outil de perçage (20, 10), la partie intérieure (20) étant solidaire en rotation de la partie extérieure (10), et un capteur (40) est agencé à une distance fixe par rapport à l'extrémité de la partie extérieure (10) et détecte le déplacement axial de la partie intérieure (20) par rapport à la partie extérieure (10), en particulier par voie capacitive, inductive, ou au moyen d'une barrière lumineuse, et le système, en cas de déplacement de la partie intérieure (20) par rapport à la partie extérieure (10) d'une valeur prédéterminée, arrête l'entraînement (50) de l'outil de perçage (20, 10), en particulier directement.

2. Dispositif de perçage selon la revendication 1, **caractérisé en ce que** le mouvement relatif de l'extrémité (28), qui dépasse vers l'arrière en direction axiale par rapport à la partie extérieure (10), de la partie intérieure (20) par rapport au capteur (40) déclenche le processus de commutation pour arrêter l'entraînement (50).

3. Dispositif de perçage selon la revendication 2, **caractérisé en ce que** l'extrémité (28) de la partie intérieure (20) est élargie radialement, en particulier sous la forme d'une collerette.

4. Dispositif de perçage selon la revendication 1, **caractérisé en ce que** le processus de commutation est déclenché par l'extrémité (28), dépassant en direction axiale vers l'arrière par rapport à la partie extérieure (10), de la partie intérieure (20), qui libère ou qui bloque un rayon de lumière qui attaque le capteur.

5. Dispositif de perçage selon l'une des revendications 1 à 4, **caractérisé en ce que** la partie intérieure (20) est réalisée sous la forme d'une tige sensiblement cylindrique.

6. Dispositif de perçage selon l'une des revendications précédentes, **caractérisé en ce que** la précontrainte axiale de la partie intérieure (10) a lieu au moyen d'un ressort (30) bandé entre une bride extérieure (24a) de la partie intérieure (20) et une bride intérieure (14a) de la partie extérieure (10).

7. Dispositif de perçage selon l'une des revendications précédentes, **caractérisé en ce que** le la partie intérieure (20) est susceptible d'être fixée dans sa situation déployée.

8. Dispositif de perçage selon l'une des revendications précédentes, **caractérisé en ce que** le capteur (40) est un capteur de Hall, qui détecte l'extrémité (28) de la partie intérieure (20).

9. Dispositif de perçage selon l'une des revendications précédentes, **caractérisé en ce que** le capteur (40) engendre un signal de capteur qui est un signal d'entrée du système et au moyen duquel le système pilote un organe de commutation, en particulier un transistor ou un relais, pour arrêter l'entraînement (50).

10. Dispositif de perçage selon l'une des revendications précédentes, **caractérisé en ce que** le capteur (40) est agencé à côté de ou axialement en arrière de l'extrémité (28) de la partie intérieure (20) dans la poignée du dispositif de perçage.

11. Dispositif de perçage selon l'une des revendications précédentes, **caractérisé en ce que** la valeur prédéterminée est susceptible d'être imposée et/ou programmée au moyen d'un élément de saisie, en particulier sous la forme d'un potentiomètre ou d'un clavier.

12. Dispositif de perçage selon l'une des revendications précédentes, **caractérisé en ce que** la partie extérieure (10) est reliée solidairement à un premier pignon conique qui engrène avec un second pignon conique, de sorte que le second pignon conique est relié à une première extrémité d'un arbre, en particulier souple, qui est relié à son autre seconde extrémité à l'entraînement électrique (50) ou un mécanisme interposé entre celles-ci.

13. Dispositif de perçage selon la revendication 12, **caractérisé en ce que** le premier pignon conique entoure la partie extérieure ou bien est fixé à l'extrémité de la partie extérieure, et le second pignon conique est monté sur la poignée ou sur une pièce agencée dans celle-ci.

14. Dispositif de perçage selon l'une des revendications précédentes, **caractérisé en ce que** le capteur (40) est un capteur de distance ou d'éloignement, qui détermine l'éloignement de l'extrémité (28) de la partie intérieure (20) par rapport au capteur (40) et qui engendre un signal correspondant et le transmet au système.

15. Dispositif de perçage selon l'une des revendications précédentes, **caractérisé en ce qu'**il est possible de déplacer par application d'une force sur la pointe (22) de la partie intérieure (20) à l'encontre de la force du ressort (30) en direction du capteur (40), en raison de quoi le système démarre l'entraînement (50).

16. Dispositif de perçage selon l'une des revendications précédentes, **caractérisé en ce que** le système de régulation (42) arrête l'entraînement dès que le capteur (40) détecte un mouvement relatif d'une taille suffisante.

17. Dispositif de perçage selon la revendication 16, **caractérisé en ce que** le mouvement relatif a lieu par un enfoncement soudain de la pointe de centrage (22), de la partie intérieure (20), qui se trouve en avant pendant le processus de perçage, en raison d'une force antagoniste extérieure qui est supérieure à la force du ressort (30).

18. Dispositif de perçage selon l'une des revendications précédentes, **caractérisé en ce que** la liaison solidaire en rotation de la partie intérieure (20) et de la partie extérieure (10) est établie
- soit par une tige de centrage (26) agencée perpendiculairement à l'axe de perçage, qui traverse la partie intérieure (10) et deux trous oblongs (16) dans la partie extérieure (10), dont la longueur détermine la course de la partie intérieure (20) par rapport à la partie extérieure (10),
- soit par des gorges et des barrettes, s'étendant radialement sur la face extérieure de la partie intérieure (20) et sur la face intérieure de la partie extérieure (10), qui peuvent être amenées en engagement mutuel par enfichage radial.
